Europäisches Patentamt

European Patent Office

Office européen des brevets

(11) Publication number: **0 385 585**
**A1**

## EUROPEAN PATENT APPLICATION

(21) Application number: **90301066.8**

(22) Date of filing: **01.02.90**

(51) Int. Cl.5 **A61F 2/36, A61F 2/30**

(30) Priority: **06.02.89 US 306946**

(43) Date of publication of application:
**05.09.90 Bulletin 90/36**

(84) Designated Contracting States:
**AT BE CH DE ES FR GB IT LI SE**

(71) Applicant: **Horowitz, Stephen M., M.D.**
**1233 York Avenue, Apt 7 I**
**New York, New York 10021(US)**

(72) Inventor: **Horowitz, Stephen M., M.D.**
**1233 York Avenue, Apt 7 I**
**New York, New York 10021(US)**

(74) Representative: **JENSEN & SON**
**8 Fulwood Place High Holborn**
**London WC1V 6HG(GB)**

(54) **A hip prosthesis and method for implanting the same.**

(57) The present invention relates to a hip prosthesis
(6) and a method of implanting the same utilizing
dual modes of fixation. The prosthesis has a proxi-
mal portion (7) which is porous coated to permit
bone ingrowth. The prosthesis also has a distal stem
portion (8) which is adapted to be affixed by cemen-
tation. In addition, there is a tapering at the junction
between the distal stem portion and the proximal
portion. The prosthesis is implanted by reaming and
broaching the femoral stem of a patient so that it can
have a prosthesis' porous portion in contact with the
bone proximally and still have channels between the
bone and the distal stem portion which are cement
filled. In addition this design also allows an improve-
ment in cementation technique by providing an im-
proved bone-cement interlock by more efficient
pressurization.

FIG.3

## A HIP PROSTHESIS AND METHOD FOR IMPLANTING THE SAME.

### BACKGROUND OF THE INVENTION

The present invention relates to a hip prosthesis and a method for implanting the same in a patient. In particular, the present invention relates to a prosthesis and method of implanting the same which depends upon two types of fixation.

Hip prosthesis are known in the art. One of the most common types of fixation techniques used for hip prosthesis is fixation by cementation by typically using polymethlymethacrylate cement. Using a surgical device called a broach, a canal is constructed in the femoral shaft of a patient which will accommodate the femoral stem of the prosthesis. However, before inserting the prosthesis but after broaching the canal, cement is poured into the canal and the stem of the prosthesis is fully inserted therein so that the entire length of the femoral stem of the prosthesis is affixed by cementation.

One of the problems associated with cement fixation of hip prosthesis is eventual failure due to aseptic loosening of the prosthesis in the patient. It has been shown that this occurs in as many as 15-20 percent of cemented hip replacements as early as 10-15 years following implantation. It has been shown that the reason for such failure is probably due to certain movements known as micromotion at the bone-cement interface which leads to the generation of polymethylmethacrylate particles which result in a foreign loosening and the eventual failure of the cemented hip prosthesis. This activity has been the subject of research and such research has been documented in an article entitled "Effects of Polymethylmethacrylate Exposure Upon Macrophages" published in the Journal of Orthopaedic Research Vol. 6, No. 6, 1988, and of which the applicant is a co-author.

In addition , other researchers have shown that improvements in cement techniques such as cementation under pressure leads to improved bone cement interlock resulting in increased longevity for the cemented prosthesis.

Another and more recent technique of fixation for a hip prosthesis is through the use of a porous ingrowth surface. With this technique, the prosthesis is porous coated to allow bone ingrowth. This technique was developed in an attempt to construct a prosthesis which would last longer than the cemented design.

At the present time, it is too soon to tell whether a porous ingrowth prosthesis will last longer than the cemented prosthesis. However, it has been shown that poor bone stock or any factor which impairs initial prosthesis fixation will lead to early micromotion at the porous/bone interface. This motion results in fibrous tissue ingrowth, rather than bone ingrowth. This lack of bone ingrowth has been implicated as a likely mode of failure of this prosthesis. Bone ingrowth into porous coated prosthesis occurs maximally during the critical period of the first six weeks of implantation. Accordingly, it would be advantageous to eliminate early micromotion so that a situation would exist allowing the maximum amount of bone ingrowth and resulting in the maximum longevity possible for the porous design. It would also be advantageous to use an additional mode of fixation in association with the porous design, then the design could be used in situations with poor bone stock.

### SUMMARY OF THE INVENTION

Hence with the foregoing in mind, it is a principal object of the present invention to provide a prosthesis and a method of implanting the same which utilizes two types of fixation and thus avoids the drawbacks associated with the aforementioned prior art proposals.

It is a further object of the invention to provide a longer lasting prosthesis, particularly for younger patients expected to outlive one or more prostheses.

It is still another object of the invention to provide a prosthesis for patients with poor bone stock such as : 1. patients on medication which weaken bone such as steroids ( which is used for arthritic patients who are frequent candidates for replacements) and 2. patients who have had prior prosthesis removed (revision situations).

The present invention provides a prosthesis having a proximal, or calcar portion which is porous coated to allow bone ingrowth and a distal stem portion which relies on polymethylmethacrylate cement for fixation.

As noted previously, bone ingrowth into porous coated prosthesis occurs maximally during the first six weeks of implementation. The present invention utilizes cement for fixation of the stem of the prosthesis thus providing a superb early stabilization and ensuring the highest quality of bone ingrowth.

The reason this stability is often difficult to obtain with most current uncemented designs is that they do not use any method other than the interference fit of the stem in bone to achieve early prosthesis stability. This interference type fixation is particularly poor when the prosthesis is to be used for a revision, or any individual with poor quality bone such as patients receiving steroids. Such a situation can occur with candidates who are arthritic patients and must take steroid medication which results in poor bone stock. In fact, these two situations are considered by many to be cotraindications to the use of uncemented designs.

Cemented prosthesis achieve maximal fixation soon after implantation. Particle generation secondary to micromotion does not really begin to become prominent until several years after prosthesis implantation.

Thus, the proposal of the present invention is that the use of cement will inhibit early micromotion and lead to a very high degree of bone ingrowth into the porous aspect of the prosthesis. In addition, it is proposed that this ingrowth will result in excellent fixation which will dramatically decrease later occurring motion at the bone-cement interface. This should impede the generation of particles and improve longevity. This improved prosthesis longevity would be particularly advantageous in younger patients, who are likely to outlive the life span of currently available prosthesis.

The prosthesis and insertion technique of the present invention provides for a better bone-cement interlock which is due to drilling a hole at the lateral aspect of the hip around the level of the lesser trochanter to drain excess cement therethrough and due to the wedge effect of the proximal portion of the prosthesis wedging into the bone. The hole and the wedge effect of the proximal portion of the prosthesis results in better pressurization of the cement into the bone and improves the interlocking between the bone and the cement.

The present invention not only provides a prosthesis in which cement is used in a porous design but also offers a new design and altered cement technique which allows both bone ingrowth and cement fixation to occur simultaneously.

## BRIEF DESCRIPTION OF THE DRAWINGS

For a fuller understanding of the nature and the objects of the invention, reference should be had to the following detailed description, taken in connection with the accompanying drawings, in which:

FIG. 1 is a perspective view of a prior art prosthesis;

FIG. 2 is a perspective view of another prior art prosthesis;

FIG. 3 is a perspective view of the prosthesis of the present invention;

FIG. 4 is a perspective view of the prosthesis of the present invention implanted within the femoral stem of a patient; and

FIGS. 5A, 5B and 5C illustrate the method of implanting the prosthesis of FIG. 3 in accordance with the technique of the present invention.

## DETAILED DESCRIPTION OF THE PREFERRED EMBODIMENT

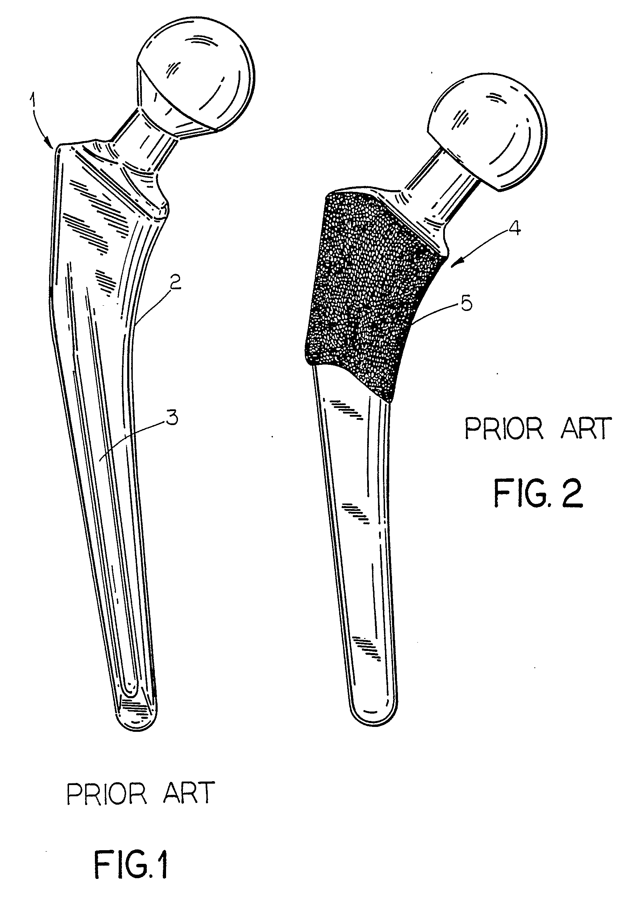

Referring now to the drawings, FIG. 1 discloses a prior art prosthesis 1 of the type which is affixed by cementation. The entire stem 2 of the prosthesis of FIG. 1 is cemented into the femoral stem of the patient. The stem 2 on such prosthesis may or may not have a longitudinal groove 3 to enhance cement fixation.

FIG. 2 disclose a prosthesis 4 having a porous contoured portion 5 to permit ingrown bone fixation when implanted in a patient.

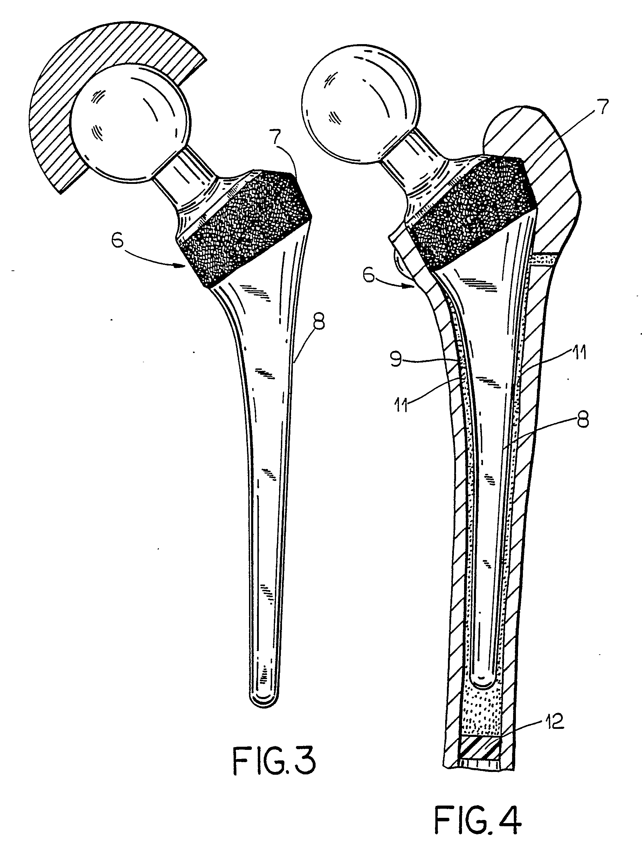

FIG.3 is the prosthesis 6 of the present invention. The prosthesis 6 has a proximal or calcar portion 7 which is porous coated for ingrown bone fixation. The prosthesis also has a distal stem portion 8 which is adapted to be cemented within the femoral stem 9 of the patient.

In FIG. 4 the prosthesis 6 is shown implanted within the patient. The proximal portion 7 of the prosthesis 6 which has the porous coating is preferably 15-20% of the length of the stem of the prosthesis as compared to 25-100 % of the stem for a conventional uncemented, porous prosthesis. The 15-20% porous length provides a sufficient stem length for cementation. Further, it is preferably if the prosthesis is manufactured at longer lengths of currently available designs or approximately 10 cm longer than this in length.

The prosthesis is tapered at the junction 10 of the proximal portion and the distal portion as shown in FIGS. 3 and 4. This differs from the typical porous prosthesis in which the stem wedges into the bone of the patient. The distal stem of prosthesis does not wedge into the bone, as it is desired to have channels 11 which are filled with cement between the distal stem portion 8 of the prosthesis and the patient's bone 9. Thus, the prosthesis has a tapering at the junction 10 to permit 2 mm channels 11 throughout the length of the distal stem 8 of the prosthesis 6.

It is understood that the present invention is not limited to the aforementioned dimensions and any other as minimal modifications that would be obvious to one skilled in the art. One such example of a minor modification would be the addition of a proximal collar.

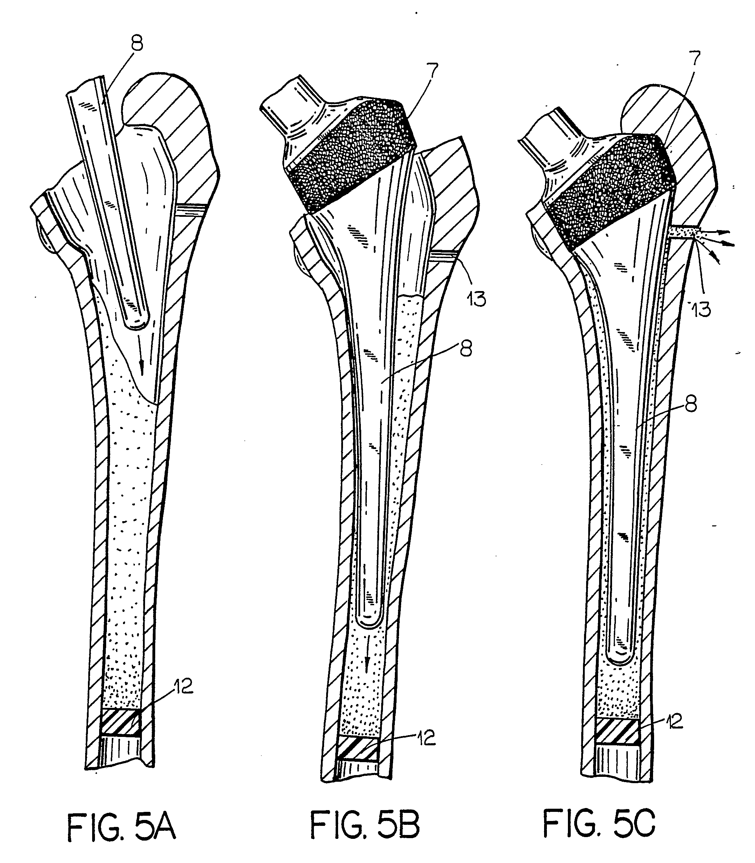

A cement retainer or plug 12 is placed below the prosthesis to prevent cement from flowing down within the patient as shown in FIG. 4 and FIGS 5A-C. This also allows enhanced pressurization of the cement.

The porous coating for the prosthesis is preferably made of cobalt-chrome or a vitallium alloy or titanium. The stem is preferably formed of either cobalt-chrome or a titanium alloy although it is understood that any other such suitable materials can be used and the invention is not limited to these specific materials.

Example

The preferred dimensions of the prosthesis include a porous coating of 1-2 cm. and a taper beginning with 0.5 cm of the end of the porous coating.

In utilizing a 9 mm uncemented prosthesis, the proximal dimension would stay the same, the porous coating would be reduced 1-2 cm, the distal stem diameter would be 8 mm (as compared to 9 mm for a conventional prosthesis) and the length of the prosthesis would be equal to the longer uncemented types (from 15-20 cm). As to the degree of tapering of the stem, the distal stem portion would be one mm. length in diameter narrower than it would be in a conventional uncemented prosthesis.

It is understood that the above dimensions are given as one example and the invention is not limited to these dimensions.

FIGS. 5A-C illustrate the method of implanting the prosthesis in a patient.

Using a reamer and a broach, conventional surgical tools, the inner part of the bone is reamed and broached to the appropriate dimensions to accommodate the contours including the tapering of the prosthesis 6.

Preferably a converted 9 mm broach would be used. Also instead of reaming to a diameter of 9 mm distally, it is preferable to over ream to 10 mm to ensure continuous 2 mm channels between the prosthesis and the bone for the cement to filled therein.

A hole 13 is then drilled approximately 1/4" in diameter at the lateral aspect of the hip around the level of the lesser trochanter as shown in FIGS. 5A-C. The hole 13 serves to permit any excess cement to be drained as therethrough.

After drilling the bone, a cement retainer or plug 12 as shown in FIGS. 5A-C is placed therein to prevent cement from flowing downward.

Cement is then injected through the neck of the femur. The level of the cement should be below the level of the hole 13 and preferably at a level of 2-3 cm. If too much cement is injected the excess amount will drain out of the hole 13. If too little cement is injected and the prosthesis is already inserted then more cement can be injected through the hole 13.

After the cement is injected, the prosthesis 6 is inserted through the neck of the femur. The proximal portion 7 of the prosthesis 6 wedges into the bone 9 and thus functions as an upper plug pressurizing any excess cement into flowing out through the hole 13. This wedge effect is yet another advantage of the present invention. There is an improved interlocking between the bone and the cement because of better pressurization of the cement into the bone due to the hole 13 and the wedge effect of the proximal portion 7 of the prosthesis 6.

I do not limit myself to any particular details of construction set forth in this specification and illustrated in the accompanying drawings, as the same refers to and sets forth only certain embodiments of the invention, and it is observed that the same may be modified without departing from the spirit and scope of the invention.

Having thus described the invention, what I claim as new and desire to be secured by Letters Patent is as follows:

**Claims**

1. A prosthesis for a hip, comprising:
a stem including an upper proximal portion having a porous coating to facilitate in-bone growth; and
a lower stem portion contoured and adapted to be affixed by cementation thereby providing dual modes of fixation upon insertion into a patient.

2. A prosthesis according to claim 1 wherein said proximal portion has a wedge-like effect against a patient's bone when inserted to provide more efficient pressurization for cement poured into and surrounding said lower stem portion of said prosthesis.

3. A prosthesis according to claim 1 wherein said proximal portion and said lower stem portion are joined at a junction and said junction is tapered.

4. A prosthesis according to claim 3 wherein said junction has a narrowing taper in the direction of said lower stem portion.

5. A prosthesis according to claim 1 wherein said proximal portion is approximately 15-20 percent of the length of the stem of said prosthesis.

6. A prosthesis according to claim 1 wherein said lower stem portion has a longitudinal groove to facilitate affixation by cementation upon insertion into a patient.

7. A prosthesis according to claim 1 wherein the stem of said prosthesis is made of cobalt-chrome material.

8. A prosthesis according to claim 1 wherein the stem of said prosthesis is made of titanium alloy.

9. A prosthesis according to claim wherein said porous coating is made of cobalt-chrome.

10. A prosthesis according to claim 1 wherein said porous material is made of a titanium alloy.

11. A method of implanting a hip prosthesis in a patient, the improvement comprising:
making a hole in the lateral aspect of a patient's hip around the level of the lesser trochanter; injecting cement into the patient's femoral stem wherein said hole drains out any excess cement thus maintaining the cement level below the level of the hole and;
inserting a hip prosthesis having a distal stem portion adapted to be affixed by the cement and a proximal portion having a porous coating to facilitate ingrown bone fixation thereby providing dual modes of fixation upon insertion into a patient.

12. A method according to claim 11 further comprising the step of broaching and overreaming the femoral stem of the patient to form channels between said distal stem of said prosthesis and the bone of the patient, said channels being cement filled after the cement is injected therein.

13. A method according to claim 11 wherein insertion of the proximal portion creates a wedge-like effect against the patient's bone and said wedge-like effect and said hole provide a more efficient pressurization for the cement poured into and surrounding the lower stem portion for cement into bone interlocking.

14. A method according to claim 11 wherein said channels are approximately 2 mm. in diameter.

15. A method according to claim 11 wherein the cement injected into the patient's femoral stem is polymethylmethacrylate cement.

16. A method according to claim 11 wherein said hole is made by drilling.

17. A method according to claim 16 wherein said hole is drilled to approximately 1/4 inch in diameter.

18. A method according to claim 11 wherein prior to injecting the cement a cement retainer is inserted within the patient and located below where the prosthesis is to be inserted so that said cement retainer prevents cement from flowing downward.

PRIOR ART

FIG.1

PRIOR ART

FIG. 2

FIG. 3

FIG. 4

FIG. 5A       FIG. 5B       FIG. 5C

European Patent Office

Application number

EP 90301066.8

# PARTIAL EUROPEAN SEARCH REPORT
which under Rule 45 of the European Patent Convention shall be considered, for the purposes of subsequent proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (Int. Cl.⁵) |
|---|---|---|---|
| Y | FR - A - 2585945 (ARMOR) <br> * figure 1; page 8, line 27 - page 9, line 27; claims 1,3 * | 1 | A61F2/36 <br> A61F2/30 |
| A | | 2,6,8, 10 | |
| Y | US - A - 4406023 (HARRIS,W.H.) <br> * column 4, line 27 - column 5, line 20; column 6, lines 45-58 * | 1 | |
| A | | 7,9 | |
| A | US - A - 4536894 (GALANTE et al.) <br> * abstract * | 1 | |
| A | US - A - 4546501 (GUSTILO et al.) <br> * abstract * | 1 | |
| A | EP - A - 0241846 (NEW YORK SOCIETY FOR THE RELIEF OF THE RUPTURED AND CRIPPLED MAINTAINING THE HOSPITAL FOR SPECIAL SURGERY THE HOSPITAL FOR SPE) <br> * figures 1,7; claims 1,3 * | 1 | TECHNICAL FIELDS SEARCHED (Int. Cl.⁵) <br><br> A61F |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-10
Claims searched incompletely: —
Claims not searched: 11-18  Article 52(4) EPC
Reason for the limitation of the search:

Method for treatment of the human or animal body by surgery

| Place of search | Date of completion of the search | Examiner |
|---|---|---|
| Berlin | 07.05.1990 | P.K. KANAL |

EPO Form 1505.1 03 82